# EUROPÄISCHE PATENTSCHRIFT

(11) **EP 3 434 207 B1**
(45) Veröffentlichungstag und Bekanntmachung des Hinweises auf die Patenterteilung: **22.09.2021**
(21) Anmeldenummer: 18184347.5
(22) Anmeldetag: 19.07.2018
(51) Int. Cl.: A61B 17/16

(54) **CHIRURGISCHES INSTRUMENT**
SURGICAL INSTRUMENT
INSTRUMENT CHIRURGICAL

(30) Priorität: 26.07.2017 DE 102017116935
(43) Veröffentlichungstag der Anmeldung: 30.01.2019
(73) Patentinhaber: Karl Storz SE & Co. KG, 78532 Tuttlingen (DE)
(72) Erfinder: Rominger, Markus, 78606 Seitingen-Oberflacht (DE); Haunschild, Karl-Heinz, 78532 Tuttlingen (DE); Schmidberger, Jochen, 78532 Tuttlingen (DE)
(74) Vertreter: Daub, Thomas

(56) Entgegenhaltungen:
- EP-A1- 1 302 168

## Beschreibung

### Stand der Technik

Die Erfindung betrifft ein chirurgisches Element nach dem Oberbegriff des Anspruchs 1 und ein Verfahren zur Demontage eines chirurgischen Instruments nach Anspruch 14.

Die Druckschrift DE 10 2006 012 754 A1 offenbart ein chirurgisches Instrument, welches zur Reinigung des Instruments zumindest teilweise zerlegbar ist.

Die Druckschrift EP 1 302 168 A1 offenbart ein chirurgisches Instrument, welches zwei Werkzeugelemente sowie eine Betätigungseinheit und ein Koppelelement aufweist, wobei das Koppelelement zur Freigabe von Betätigungselementen der Betätigungseinheit von einer ersten Stellung in eine zweite Stellung überführbar ist.

Die Aufgabe der Erfindung besteht insbesondere darin, eine gattungsgemäße Vorrichtung mit verbesserten Eigenschaften hinsichtlich einer Zerlegbarkeit, insbesondere zur Reinigung, bereitzustellen. Die Aufgabe wird erfindungsgemäß durch die Merkmale der Ansprüche 1 und 14 gelöst, während vorteilhafte Ausgestaltungen und Weiterbildungen der Erfindung den Unteransprüchen entnommen werden können.

### Offenbarung der Erfindung

Die Erfindung betrifft ein chirurgisches Instrument mit zumindest einer Werkzeugeinheit, welche ein erstes Werkzeugelement, zumindest ein zweites Werkzeugelement und zumindest ein mit dem zweiten Werkzeugelement verbundenes Koppelelement aufweist, welche gemeinsam relativ zu dem ersten Werkzeugelement entlang zumindest einer Verschiebeachse verschiebbar gelagert sind, und mit einer vorzugsweise manuell betätigbaren Betätigungseinheit, welche zumindest ein Betätigungselement aufweist, welches in zumindest einer ersten Stellung des Koppelelements relativ zum zweiten Werkzeugelement mit dem Koppelelement gekoppelt ist, wobei das Koppelelement in zumindest einer zweiten Stellung relativ zum zweiten Werkzeugelement das Betätigungselement freigibt.

Unter einem "chirurgischen Instrument" soll insbesondere ein Instrument verstanden werden, welches für schneidende und/oder scherende und/oder klemmende und/oder fassende und/oder weitere einem Fachmann bekannte Eingriffe, insbesondere in einem menschlichen Körper, vorgesehen ist. Insbesondere kann das chirurgische Instrument als Knochenstanze und/oder als Ohrzange und/oder als medizinische Gewebestanze und/oder als Fasszange und/oder als Präparierklemme und/oder als Spekulum und/oder als Retraktore ausgebildet sein und/oder insbesondere im Bereich der Oto-Rhino-Laryngologie zur Durchführung von Biopsien und/oder dergleichen verwendet werden. Unter einer "Werkzeugeinheit" soll insbesondere eine Einheit verstanden werden, welche in zumindest einem Betriebszustand in einem direkten Kontakt mit zumindest einem zu bearbeitenden Objekt, insbesondere menschlichem Gewebe, steht.

Unter einem "Koppelelement" soll insbesondere ein Element verstanden werden, welches in zumindest einem Betriebszustand ein erstes Element mit zumindest einem weiteren Element zumindest hinsichtlich einer Kraftwirkung verbindet. Der Ausdruck "verschiebbar gelagert" soll hier insbesondere eine Lagerung einer Einheit und/oder eines Elements definieren, wobei die Einheit und/oder das Element, insbesondere entkoppelt von einer elastischen Verformung der Einheit und/oder des Elements, eine Bewegungsfreiheit entlang zumindest einer Achse von mehr als 2 mm, bevorzugt von mehr als 4 mm und besonders bevorzugt von mehr als 6 mm aufweist. Unter einer "Verschiebeachse" soll insbesondere eine Achse verstanden werden, welche vorteilhaft zumindest teilweise entlang einer translatorischen Bewegung, insbesondere des zweiten Werkzeugelements, gelegt ist.

Darunter, dass zumindest ein erstes Element mit zumindest einem weiteren Element "verbunden" ist, soll insbesondere verstanden werden, dass das erste Element vorteilhaft über zumindest einen Kraftschluss und/oder zumindest einen Formschluss mit dem weiteren Element verbunden ist, beispielsweise über eine Vernietung und/oder Rastverbindung und/oder eine Nut-Feder-Verbindung und/oder eine Klemmverbindung und/oder eine weitere dem Fachmann als sinnvoll erscheinende Verbindung, und/oder stoffschlüssig mit dem weiteren Element verbunden sind, beispielsweise durch einen Schweißprozess, einen Klebeprozess, einen Anspritzprozess und/oder einen anderen dem Fachmann als sinnvoll erscheinenden Prozess, wobei die Verbindung vorzugsweise zumindest teilweise eine Bewegung des ersten Elements relativ zum weiteren Element zulässt, wobei insbesondere zumindest ein Freiheitsgrad der Bewegung durch die Verbindung beschränkt ist.

Unter einer "Betätigungseinheit" soll insbesondere eine Einheit verstanden werden, welche zu einer Betätigung der Werkzeugeinheit durch einen Nutzer, insbesondere einen Arzt und/oder einen Arzthelfer, vorgesehen ist. Darunter dass die Betätigungseinheit "manuell" betätigbar ist, soll insbesondere verstanden werden, dass die Betätigungseinheit in zumindest einem Betriebszustand von einem Nutzer, insbesondere mittels der eigenen Kraft des Nutzers, vorteilhaft einer Hand des Nutzers, betätigt wird. Alternativ oder zusätzlich kann die Betätigungseinheit in zumindest einem Betriebszustand zumindest teilweise autonom, vorzugsweise mittels einer aktiven Antriebseinheit, beispielsweise einem Motor, betätigt werden. Vorzugsweise weist die Betätigungseinheit ein erstes Griffelement und zumindest ein zweites Griffelement auf, welche insbesondere zur manuellen Betätigung des Werkzeugelements vorgesehen sind und hierzu insbesondere anfassbar sind. Besonders bevorzugt ist das erste Griffelement relativ zum zweiten Griffelement beweglich, insbesondere schwenkbar gelagert. Insbesondere ist das erste Griffelement vorzugsweise fest und besonders bevorzugt einteilig mit dem Betätigungselement verbunden. Insbesondere ist die Betätigungseinheit zumindest teilweise mit dem zweiten Werkzeugelement verbunden. Vorzugsweise ist das zweite Griffelement fest und besonders bevorzugt einteilig mit dem zweiten Werkzeugelement verbunden.

Unter "einteilig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling.

Vorzugsweise weist das Koppelelement eine Aussparung auf, welche zumindest teilweise dazu vorgesehen ist, das Betätigungselement aufzunehmen und/oder zu führen. Ferner weist das Koppelelement vorteilhaft eine Kraftübertragungsfläche auf, welche insbesondere einen direkten Kontakt mit dem Betätigungselement aufnimmt, insbesondere zu einer Kraftübertragung von der Betätigungseinheit zur Werkzeugeinheit. Darunter, dass das Koppelelement vom Betätigungselement "freigegeben" ist, soll insbesondere verstanden werden, dass insbesondere ein Kontakt zwischen dem Koppelelement und dem Betätigungselement aufgehoben ist, wobei vorteilhaft das Koppelelement und das damit verbundene zweite Werkzeugelement von der Betätigungseinheit und/oder dem ersten Koppelelement lösbar ist.

Unter "vorgesehen" soll insbesondere speziell ausgelegt und/oder speziell ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt.

Durch die erfindungsgemäße Ausgestaltung können verbesserte Eigenschaften hinsichtlich einer Zerlegbarkeit erreicht werden. Insbesondere kann das chirurgische Instrument vorteilhaft nach einem Einsatz für einen Reinigungsprozess schnell und einfach in seine wesentlichen Einzelteile und/oder Bauteile zerlegt werden. Ferner können vorteilhaft die Betätigungseinheit und/oder die Werkzeugeinheit, vorzugsweise deren Oberflächen, Hohlräume, Lumen und/oder Öffnungen, sicher mittels maschineller und/oder manueller Reinigung erreicht werden. Hierdurch kann vorteilhaft ein Infektionsrisiko, insbesondere innerhalb eines lebenden Organismus, vermindert werden. Des Weiteren kann vorteilhaft das erste Werkzeugelement und/oder die Betätigungseinheit mit dem zweiten Werkzeugelement, insbesondere nach erfolgter Reinigung, schnell und effizient zusammengesetzt werden. Hierdurch können vorteilhaft eine Zuverlässigkeit und/oder vorzugsweise eine Patientensicherheit gesteigert werden, wodurch insbesondere ein Verletzungsrisiko für Patienten minimiert werden kann.

Das Koppelelement ist erfindungsgemäß um zumindest eine Schwenkachse schwenkbar an dem zweiten Werkzeugelement gelagert. Vorzugsweise liegt zwischen der ersten Stellung und der zweiten Stellung des Koppelelements relativ zum zweiten Werkzeugelement ein Drehwinkel von zumindest 5°, vorzugsweise von zumindest 15° und besonders vorteilhaft von zumindest 30° und insbesondere von maximal 90°, vorzugsweise von maximal 75° und besonders vorteilhaft von maximal 45°. Hierdurch kann vorteilhaft eine sichere, einfache und schnelle Entriegelung ermöglicht werden.

Vorteilhaft ist die Schwenkachse zumindest im Wesentlichen senkrecht zur Verschiebeachse ausgerichtet. Der Ausdruck "zumindest im Wesentlichen senkrecht" soll hier insbesondere eine Ausrichtung einer Richtung relativ zu einer Bezugsrichtung definieren, wobei die Richtung und die Bezugsrichtung, insbesondere in einer Ebene betrachtet, einen Winkel von 90° einschließen und der Winkel eine maximale Abweichung von insbesondere kleiner als 8°, vorteilhaft kleiner als 5° und besonders vorteilhaft kleiner als 2° aufweist. Hierdurch kann eine vorteilhafte Haptik erreicht werden. Zudem kann eine sichere und insbesondere nicht selbstlösende Verriegelung bereitgestellt werden.

In einer weiteren Ausgestaltung wird vorgeschlagen, dass die Schwenkachse zumindest im Wesentlichen parallel zu einer Haupterstreckungsebene der Betätigungseinheit ausgerichtet ist. Unter einer "Haupterstreckungsebene" eines Objekts soll insbesondere eine Ebene verstanden werden, welche parallel zu einer größten Seitenfläche eines kleinsten gedachten Quaders ist, welcher das Objekt gerade noch vollständig umschließt, und insbesondere durch den Mittelpunkt des Quaders verläuft. Unter "zumindest im Wesentlichen parallel" soll hier insbesondere eine Ausrichtung einer Richtung relativ zu einer Bezugsrichtung, insbesondere in einer Ebene, verstanden werden, wobei die Richtung gegenüber der Bezugsrichtung eine Abweichung insbesondere kleiner als 8°, vorteilhaft kleiner als 5° und besonders vorteilhaft kleiner als 2° aufweist. Insbesondere ist dadurch das Koppelelement seitlich relativ zur Betätigungseinheit schwenkbar. Vorteilhaft ist die Schwenkachse zumindest im Wesentlichen parallel zu einer Haupterstreckungsgeraden des ersten Griffelements. Unter einer "Haupterstreckungsgeraden" eines Objekts soll dabei insbesondere eine Gerade verstanden werden, welche parallel zu einer längsten Kante eines kleinsten gedachten Quaders verläuft, welcher das Objekt gerade noch vollständig umschließt. Alternativ kann die Schwenkachse zumindest im Wesentlichen senkrecht zur Haupterstreckungsebene der Betätigungseinheit angeordnet sein, wodurch insbesondere das Koppelelement nach oben relativ zur Betätigungseinheit schwenkbar ist. Hierdurch kann eine besonders einfach bedienbare und sichere Verriegelung der Werkzeugeinheit bereitgestellt werden.

Zudem wird vorgeschlagen, dass die Werkzeugeinheit zumindest eine Gleitlagereinheit aufweist, welche in der ersten Stellung das zweite Werkzeugelement mit dem ersten Werkzeugelement verbindet und das zweite Werkzeugelement an dem ersten Werkzeugelement gleitend lagert. Vorzugsweise weist die Gleitlagereinheit zumindest ein mit dem ersten Werkzeugelement vorzugsweise einstückig verbundenes erstes Gleitlagerelement und zumindest ein mit dem zweiten Werkzeugelement vorzugsweise einstückig verbundenes, korrespondierendes zweites Gleitlagerelement auf, wobei die Gleitlagerelemente insbesondere mittels zumindest eines Formschlusses aneinander gleitend ausgebildet sind. Hierdurch kann vorteilhaft ein stabiles und sicheres Verschieben des zweiten Werkzeugelements relativ zum ersten Werkzeugelement erreicht werden.

Weiterhin wird vorgeschlagen, dass in der zweiten Stellung eine von der Gleitlagereinheit bereitgestellte Lagerung aufhebbar und das zweite Werkzeugelement vom ersten Werkzeugelement lösbar ist. Insbesondere weist die Gleitlagereinheit zumindest zum Lösen des zweiten Werkzeugelements eine Öffnung und/oder eine Aussparung auf, durch welche vorteilhaft eine Lagerung, zumindest eines Gleitlagerelements, aufhebbar ist. Insbesondere ist durch ein Verschieben des zweiten Werkzeugelements relativ zum ersten Werkzeugelement, insbesondere entlang der Verschiebeachse, eine Lagerung aufhebbar. Hierdurch kann vorteilhaft eine stabile Werkzeugeinheit bereitgestellt werden, wobei insbesondere ein einfaches Lösen ermöglicht wird.

Vorteilhaft ist, dass das zweite Werkzeugelement insbesondere in der zweiten Stellung durch eine Bewegung entlang der Verschiebeachse von dem ersten Werkzeugelement lösbar ist. Vorzugsweise ist die Bewegung des zweiten Werkzeugelements zum Lösen des zweiten Werkzeugelements entlang der Verschiebeachse zumindest teilweise in Richtung des Betätigungselements gerichtet. Hierdurch kann ein Lösen des zweiten Werkzeugelements vom ersten Werkzeugelement weiter vorteilhaft vereinfacht werden.

In einer besonders bevorzugten Ausführung der Erfindung wird vorgeschlagen, dass die Gleitlagereinheit das Koppelelement in der ersten Stellung gleitend am ersten Werkzeugelement lagert. Insbesondere weist die Gleitlagereinheit ein mit dem Koppelelement insbesondere fest und vorzugsweise einstückig verbundenes Führungsteil auf, welches zu einer Führung des Koppelelements am ersten Werkzeugelement vorgesehen ist. Insbesondere kann das Führungsteil zusätzlich zu einer Stabilisierung des zweiten Werkzeugelements vorgesehen sein. Vorzugsweise ist das Führungsteil als eine Feder ausgebildet und dazu vorgesehen, in eine Nut der Gleitlagereinheit, wobei die Nut insbesondere fest und vorzugsweise einstückig mit dem ersten Werkzeugelement verbunden ist, einzugreifen. Vorzugsweise ist die Nut zusätzlich dazu vorgesehen, eine Führung des Betätigungselements bereitzustellen. Insbesondere weist das zweite Werkzeugelement eine Öffnung auf, welche insbesondere dazu vorgesehen ist, das Führungsteil bei einem Schwenken des Koppelelements relativ zum zweiten Werkzeugelement freizugeben. Hierdurch kann vorteilhaft eine einfache und flexible Konstruktion bereitgestellt werden. Ferner wird vorgeschlagen, dass das chirurgische Instrument zumindest eine Verriegelungseinheit aufweist, welche das Koppelelement in der ersten Stellung verriegelt. Vorzugsweise verhindert die Verriegelungseinheit zumindest teilweise ein Lösen des ersten Werkzeugelements von dem zweiten Werkzeugelement. Alternativ oder zusätzlich kann die Verriegelungseinheit zumindest teilweise ein Rastelement aufweisen, welches insbesondere dazu vorgesehen sein kann, das Koppelelement zumindest in der ersten Stellung einzurasten. Insbesondere ist das Rastelement dabei zumindest teilweise als Nocke und/oder Ecke und/oder Kontur ausgebildet, welche insbesondere fest und vorzugsweise einstückig mit dem Koppelelement und/oder dem zweiten Werkzeugelement verbunden ist. Hierdurch kann eine Zuverlässigkeit gesteigert werden, wodurch insbesondere eine hohe Patientensicherheit gewährleistet werden kann.

In einer besonders bevorzugten Ausgestaltung der Erfindung wird vorgeschlagen, dass die Verriegelungseinheit zumindest ein relativ zur Werkzeugeinheit schwenkbar gelagertes Verriegelungselement aufweist. Der Ausdruck "schwenkbar gelagert" soll hier insbesondere eine Lagerung einer Einheit und/oder eines Elements definieren, wobei die Einheit und/oder das Element, insbesondere entkoppelt von einer elastischen Verformung der Einheit und/oder des Elements, eine Bewegungsmöglichkeit um zumindest eine Achse um einen Winkel größer als 10°, bevorzugt größer als 40° und besonders bevorzugt größer als 70° aufweist. Hierdurch kann vorteilhaft eine Verriegelung weiter verbessert werden. Ferner kann eine zusätzliche Stabilisierung der Werkzeugeinheit in der ersten Stellung erreicht werden.

Weiterhin wird vorgeschlagen, dass das Verriegelungselement in zumindest einem Betriebszustand zumindest teilweise ein Schwenken des Koppelelements relativ zum zweiten Werkzeugelement verhindert. Besonders bevorzugt wird ein Schwenken in der ersten Stellung verhindert. Alternativ oder zusätzlich weist die Verriegelungseinheit zumindest ein Einrastelement auf, welches das Koppelelement in der zweiten Stellung einrastet. Hierdurch kann vorteilhaft eine Bedienbarkeit weiter vereinfacht werden.

Ferner wird vorgeschlagen, dass das Verriegelungselement insbesondere schwenkbar an dem ersten Werkzeugelement gelagert ist. Bevorzugt wird das Verriegelungselement mittels einer Nietverbindung und/oder einer Schraubverbindung und/oder einer Klemmverbindung und/oder einer Rastverbindung und/oder einer weiteren einem Fachmann als sinnvoll erscheinenden Verbindung mit dem ersten Werkzeugelement verbunden. Ferner ist insbesondere die Schwenkachse des Verriegelungselements vorteilhaft senkrecht zu der Schwenkachse des Koppelelements und/oder senkrecht zur Haupterstreckungsebene der Betätigungseinheit. Vorzugsweise ist das Verriegelungselement lediglich dazu vorgesehen eine Drehbewegung um die Schwenkachse auszuführen. Hierdurch kann Verriegelungseinheit mit vorteilhaft hoher Stabilität und/oder Zuverlässigkeit bereitgestellt werden.

In einer weiteren Ausgestaltung der Erfindung wird vorgeschlagen, dass das Verriegelungselement insbesondere schwenkbar an dem Koppelelement gelagert ist. Vorteilhaft ist das Verriegelungselement mittels einer Rastverbindung und/oder einer Vernietung und/oder eine Klemmverbindung und/oder einer weiteren einem Fachmann als sinnvoll erscheinenden Verbindung mit dem Koppelelement verbunden. Insbesondere ist das Verriegelungselement zusammen mit dem Koppelelement und/oder dem zweiten Werkzeugelement in der ersten Stellung entlang der Verschiebeachse verschiebbar. Vorteilhaft ist eine Haupterstreckungsgerade des Verriegelungselements in der ersten Stellung zumindest im Wesentlichen parallel zu der Verschiebeachse. Vorzugsweise umschließt das Verriegelungselement zumindest teilweise das Betätigungselement. Ferner ist zumindest in der ersten Stellung insbesondere die Schwenkachse des Verriegelungselements vorteilhaft senkrecht zu der Schwenkachse des Koppelelements und/oder senkrecht zur Haupterstreckungsebene der Betätigungseinheit. Vorzugsweise ist das Verriegelungselement dazu vorgesehen, sich zumindest teilweise um die Schwenkachse des Koppelelements, vorteilhaft mit dem Kopplungselement, zu drehen. Hierdurch kann ein vorteilhaft bedienerfreundliches Verriegelungselement bereitgestellt werden.

Ferner wird vorgeschlagen, dass die Gleitlagereinheit zumindest teilweise einstückig mit der Verriegelungseinheit ausgebildet ist. Darunter, dass zwei Einheiten "zumindest teilweise einstückig" miteinander ausgebildet sind, soll in diesem Zusammenhang insbesondere verstanden werden, dass die beiden Einheiten sich wenigstens ein Element teilen. Vorzugsweise weist die Verriegelungseinheit zumindest ein Element auf, welches auch Teil der Gleitlagereinheit ist und insbesondere zumindest eine Gleitlagerfunktion bereitstellt. Hierdurch kann vorteilhaft eine Stabilität verbessert werden. Zudem kann eine Bauteileanzahl reduziert werden.

Weiterhin wird vorgeschlagen, dass das Verriegelungselement zumindest eine Gleitführungsfläche für das Koppelelement bereitstellt. Insbesondere ist die Gleitführungsfläche einstückig mit dem Verriegelungselement ausgebildet. Vorteilhaft ist die Gleitführungsfläche zumindest teilweise im direkten Kontakt mit dem ersten Werkzeugelement, dem zweiten Werkzeugelement und/oder dem Koppelelement. Vorzugsweise ist die Gleitführungsfläche dazu vorgesehen, ein axiales Verschieben entlang der Verschiebeachse des Koppelelements weiterhin zu stabilisieren. Hierdurch kann eine Stabilität weiter vorteilhaft gesteigert werden.

Ferner betrifft die Erfindung ein Verfahren zur Demontage eines chirurgischen Instruments mit zumindest einer Werkzeugeinheit, welche ein erstes Werkzeugelement, zumindest ein zweites Werkzeugelement und zumindest ein mit dem zweiten Werkzeugelement verbundenes Koppelelement aufweist, welche gemeinsam relativ zu dem ersten Werkzeugelement entlang zumindest einer Verschiebeachse verschiebbar gelagert sind, und mit einer vorzugsweise manuell betätigbaren Betätigungseinheit, welche zumindest ein Betätigungselement aufweist, welches in zumindest einer ersten Stellung des Koppelelements relativ zum zweiten Werkzeugelement mit dem Koppelelement gekoppelt ist, wobei das Koppelelement um zumindest eine Schwenkachse schwenkbar an dem zweiten Werkzeugelement gelagert ist und wobei das Koppelelement von der ersten Stellung in zumindest eine zweite Stellung relativ zum zweiten Werkzeugelement zur Freigabe des Betätigungselements überführt wird.

Durch die erfindungsgemäße Ausgestaltung können verbesserte Eigenschaften hinsichtlich einer Zerlegbarkeit erreicht werden. Insbesondere kann das chirurgische Instrument vorteilhaft nach einem Einsatz für einen Reinigungsprozess schnell und einfach in seine wesentlichen Einzelteile und/oder Bauteile zerlegt werden. Ferner können vorteilhaft die Betätigungseinheit und/oder die Werkzeugeinheit, vorzugsweise deren Oberflächen, Hohlräume, Lumen und/oder Öffnungen, sicher mittels maschineller und/oder manueller Reinigung erreicht werden. Hierdurch kann vorteilhaft ein Infektionsrisiko, insbesondere innerhalb eines lebenden Organismus, vermindert werden. Des Weiteren kann vorteilhaft das erste Werkzeugelement und/oder die Betätigungseinheit mit dem zweiten Werkzeugelement, insbesondere nach erfolgter Reinigung, schnell und effizient zusammengesetzt werden. Hierdurch kann vorteilhaft eine Zuverlässigkeit gesteigert werden, wodurch insbesondere ein Verletzungsrisiko für Patienten minimiert werden kann.

Das chirurgische Instrument soll hierbei nicht auf die oben beschriebene Anwendung und Ausführungsform beschränkt sein. Insbesondere kann das chirurgische Instrument zu einer Erfüllung einer hierin beschriebenen Funktionsweise eine von einer hierin genannten Anzahl von einzelnen Elementen, Bauteilen und Einheiten abweichende Anzahl aufweisen.

### Zeichnungen

Weitere Vorteile ergeben sich aus der folgenden Zeichnungsbeschreibung. In den Zeichnungen sind drei Ausführungsbeispiele der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: ein chirurgisches Instrument, welches eine Werkzeugeinheit mit einem ersten Werkzeugelement, einem zweiten Werkzeugelement und einem Koppelelement aufweist, in einem montierten Zustand, in welchem sich das Koppelelement relativ zum zweiten Werkzeugelement in einer ersten Stellung befindet,
- Fig. 2: das chirurgische Instrument in einer zweiten Stellung des Koppelelements relativ zum zweiten Werkzeugelement,
- Fig. 3: eine Verriegelungseinheit des chirurgischen Instruments in der zweiten Stellung in einem entriegelten Zustand des Koppelelements,
- Fig. 4: einen Teil des chirurgischen Instruments umfassend das erste Werkzeugelement,
- Fig. 5: das zweite Werkzeugelement und das Koppelelement in der ersten Stellung,
- Fig. 6: ein alternatives chirurgisches Instrument,
- Fig. 7: einen Teil des chirurgischen Instruments aus Fig. 6 umfassend ein erstes Werkzeugelement einer Werkzeugeinheit,
- Fig. 8: ein zweites Werkzeugelement und ein Koppelelement der Werkzeugeinheit des chirurgischen Instruments aus Fig. 6 in einer ersten Stellung des Koppelelements relativ zum zweiten Werkzeugelement,
- Fig. 9: ein weiteres chirurgisches Instrument,
- Fig. 10: das chirurgische Instrument aus Fig. 9 in einer zweiten Stellung eines Koppelelements relativ zu einem zweiten Werkzeugelement einer Werkzeugeinheit des chirurgischen Instruments und
- Fig. 11: das chirurgische Instrument aus Fig. 9 in einem demontierten Zustand.

### Beschreibung der Ausführungsbeispiele

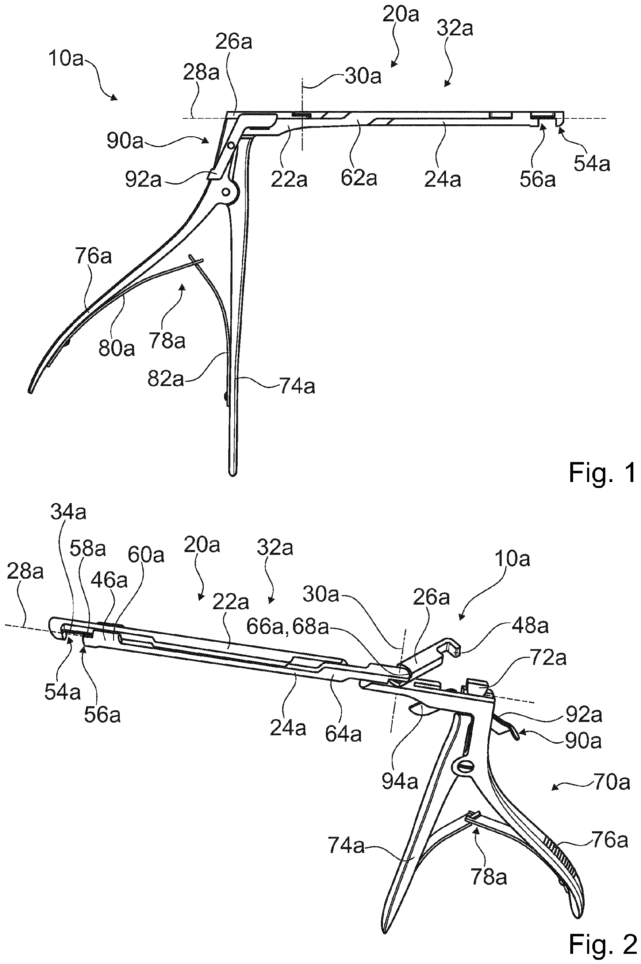

Die Figur 1 zeigt eine isometrische Darstellung eines chirurgischen Instruments 10a in einem montierten Zustand. Das chirurgische Instrument 10a ist für Eingriffe in einem menschlichen Körper vorgesehen. Das chirurgische Instrument 10a ist als medizinische Gewebestanze ausgebildet.

Das chirurgische Instrument 10a weist eine Werkzeugeinheit 20a auf. Die Werkzeugeinheit 20a weist ein erstes Werkzeugelement 22a auf. Die Werkzeugeinheit 20a weist ein zweites Werkzeugelement 24a auf. Das zweite Werkzeugelement 24a ist im montierten Zustand relativ zum ersten Werkzeugelement 22a translatorisch entlang einer Verschiebeachse 28a bewegbar gelagert. Die Werkzeugeinheit 20a weist ein erstes Schneideelement 54a auf. Die Werkzeugeinheit 20a weist zumindest ein zweites Schneideelement 56a auf. Das erste Werkzeugelement 22a weist das erste Schneideelement 54a auf. Das erste Werkzeugelement 22a ist einteilig mit dem ersten Schneideelement 54a verbunden. Das zweite Werkzeugelement 24a weist das zweite Schneideelement 56a auf. Das zweite Werkzeugelement 24a ist einteilig mit dem zweiten Schneideelement 56a verbunden. Das zweite Schneideelement 56a ist im montierten Zustand zusammen mit dem zweiten Werkzeugelement 24a entlang der Verschiebeachse 28a relativ zum ersten Schneideelement 54a bewegbar. Das erste Schneideelement 54a und das zweite Schneideelement 56a sind als Stanzschneiden ausgebildet. Das erste Schneideelement 54a ist relativ zum zweiten Schneideelement 56a zumindest im Wesentlichen parallel angeordnet. Das erste Schneideelement 54a weist zumindest im Wesentlichen eine senkrechte Anordnung relativ zum ersten Werkzeugelement 22a auf.

Das chirurgische Instrument 10a weist eine manuell betätigbare Betätigungseinheit 70a auf. Das erste Schneideelement 54a und das zweite Schneideelement 56a sind zumindest im Wesentlichen in Richtung der Betätigungseinheit 70a geöffnet. Die Betätigungseinheit 70a ist dazu vorgesehen die Werkzeugeinheit 20a zu betätigen. Die Betätigungseinheit 70a ist dazu vorgesehen, das zweite Werkzeugelement 24a im montierten Zustand relativ zum ersten Werkzeugelement 22a zu verschieben. Die Betätigungseinheit 70a weist ein erstes Griffelement 74a auf. Die Betätigungseinheit 70a weist ein zweites Griffelement 76a auf. Das erste Griffelement 74a und das zweite Griffelement 76a sind zueinander drehbar angeordnet. Das erste Griffelement 74a und das zweite Griffelement 76a sind mittels eines Bolzens verbunden.

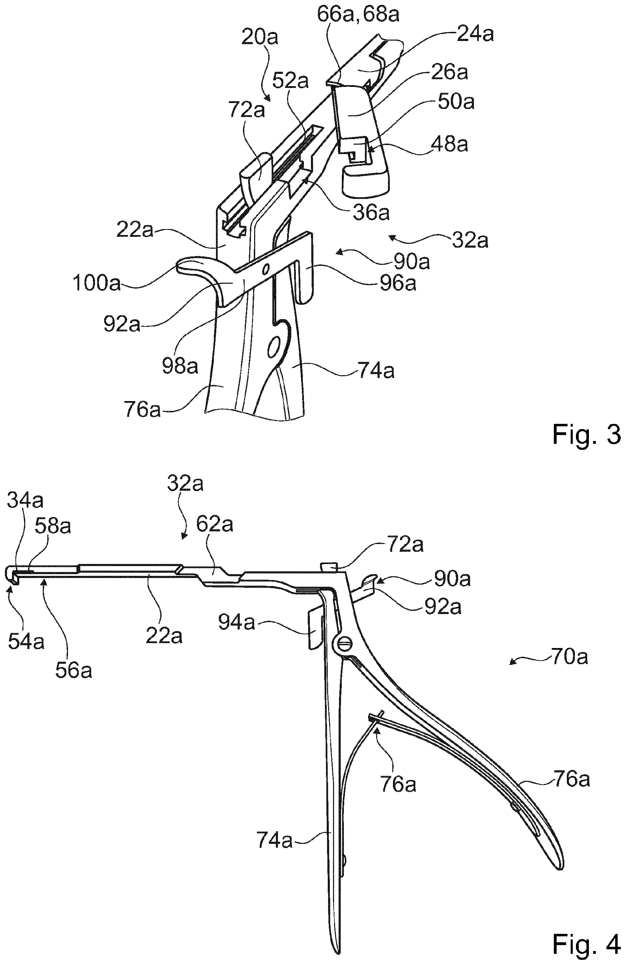

Das erste Griffelement 74a ist mittels einer Federeinheit 78a der Betätigungseinheit 70a mit dem zweiten Griffelement 76a verbunden. Die Federeinheit 78a ist dazu vorgesehen, einer Betätigung der Griffelemente 74a, 76a entgegenzuwirken und diese in eine Ausgangsstellung zurückzuführen. Die Federeinheit 78a weist ein erstes Federelement 80a auf. Die Federeinheit 78a weist ein zweites Federelement 82a auf. Die Federeinheit 78a ist bei einer Demontage der Betätigungseinheit 70a zumindest teilweise in ein erstes Federelement 80a und in ein zweites Federelement 82a teilbar. Das erste Federelement 80a ist mittels einer Formschlussverbindung mit dem zweiten Federelement 82a verbunden. Die Betätigungseinheit 70a ist zumindest teilweise einstückig mit dem ersten Werkzeugelement 22a verbunden. Das zweite Griffelement 76a ist einstückig mit dem ersten Werkzeugelement 22a verbunden (vgl. Fig. 2 und 4).

Die Betätigungseinheit 70a weist ein Betätigungselement 72a auf (vgl. Fig. 2, 3 und 4). Das Betätigungselement 72a ist einteilig mit dem ersten Griffelement 74a verbunden. Das Betätigungselement 72a ist dazu vorgesehen, das zweite Werkzeugelement 24a entlang der Verschiebeachse 28a zu bewegen. Das Betätigungselement 72a ist dazu vorgesehen, ein Koppelelement 26a der Werkzeugeinheit 20a entlang der Verschiebeachse 28a zu bewegen (vgl. Fig. 1, 2 und 3). Das zweite Werkzeugelement 24a ist mit dem Koppelelement 26a verbunden. Das zweite Werkzeugelement 24a ist mit dem Koppelelement 26a vernietet. Das Koppelelement 26a ist im montierten Zustand gemeinsam mit dem zweiten Werkzeugelement 24a relativ zum ersten Werkzeugelement 22a translatorisch entlang der Verschiebeachse 28a beweglich gelagert. Das Koppelelement 26a ist um zumindest eine Schwenkachse 30a schwenkbar an dem zweiten Werkzeugelement 24a gelagert. Die Schwenkachse 30a ist zumindest im Wesentlichen senkrecht zur Verschiebeachse 28a ausgebildet. Die Schwenkachse 30a ist zumindest im Wesentlichen parallel zu einer Haupterstreckungsebene der Betätigungseinheit 70a angeordnet. Die Schwenkachse 30a ist zumindest im Wesentlichen parallel zu einer Haupterstreckungsgeraden des zweiten Griffelements 76a.

In zumindest einer ersten Stellung des Koppelelements 26a relativ zum zweiten Werkzeugelement 24a ist das Betätigungselement 72a mit dem Koppelelement 26a gekoppelt (vgl. Fig. 1). Das Koppelelement 26a gibt in zumindest einer zweiten Stellung relativ zum zweiten Werkzeugelement 24a das Betätigungselement 72a frei (vgl. Fig. 2 und 3). Das zweite Werkzeugelement 24a ist in dieser Stellung vom ersten Werkzeugelement 22a lösbar. Das Koppelelement 26a ist in der zweiten Stellung nach außen geschwenkt. Das Koppelelement 26a ist mittels eines Einrastelements 66a in der zweiten Stellung einrastbar. Das zweite Werkzeugelement 24a weist zumindest teilweise das Einrastelement 66a auf. Das zweite Werkzeugelement 24a stellt zumindest eine Kontur 68a bereit. Die Kontur 68a bildet das Einrastelement 66a. Die Kontur 68a ist dazu vorgesehen ein Einrasten des Koppelelements 26a in der zweiten Stellung zu ermöglichen.

Das Koppelelement 26a weist eine Aussparung 48a auf. Die Aussparung 48a ist dazu vorgesehen, das Betätigungselement 72a aufzunehmen. Das Koppelelement 26a weist eine Kraftübertragungsfläche 50a auf. Die Kraftübertragungsfläche 50a ist dazu vorgesehen, einen direkten Kontakt mit dem Betätigungselement 72a aufzunehmen. Die Kraftübertragungsfläche 50a ist zur direkten Kraftübertragung vom Betätigungselement 72a zum Koppelelement 26a vorgesehen.

Das chirurgische Instrument 10a weist zumindest eine Verriegelungseinheit 90a auf, welche das Koppelelement 26a in der ersten Stellung verriegelt. Die Verriegelungseinheit 90a weist ein Verriegelungselement 92a auf. Das Verriegelungselement 92a ist zumindest relativ zur Werkzeugeinheit 20a schwenkbar gelagert. Das Verriegelungselement 92a ist an dem ersten Werkzeugelement 22a gelagert (vgl. Fig. 3). Das Verriegelungselement 92a ist mit dem ersten Werkzeugelement 22a vernietet. Das Verriegelungselement 92a stellt zumindest eine Gleitführungsfläche 94a für das Koppelelement 26a bereit. Das Verriegelungselement 92a weist zumindest einen Schenkel 96a, 98a, 100a auf. Das Verriegelungselement 92a weist drei Schenkel 96a, 98a, 100a auf. Ein erster Schenkel 96a des Verriegelungselements 92a ist zumindest teilweise als Gleitführungsfläche 94a ausgebildet. Ein zweiter Schenkel 98a ist mit dem ersten Werkzeugelement 22a verbunden. Der erste Schenkel 96a und der zweite Schenkel 98a schließen einen Winkel zwischen 90° und 120° ein. Ein dritter Schenkel 100a ist zumindest teilweise senkrecht zu dem ersten Schenkel 96a angeordnet. Eine Haupterstreckungsebene des dritten Schenkels 100a ist zumindest teilweise senkrecht zu der Haupterstreckungsebene der Betätigungseinheit 70a. Der dritte Schenkel 100a ist dazu vorgesehen, das Verriegelungselement 92a in einem verriegelten Zustand zu stabilisieren (vgl. Fig. 3).

Die Werkzeugeinheit 20a weist zumindest eine Gleitlagereinheit 32a auf. Die Gleitlagereinheit 32a ist dazu vorgesehen, in der ersten Stellung das zweite Werkzeugelement 24a mit dem ersten Werkzeugelement 22a zu verbinden. Das zweite Werkzeugelement 24a ist mittels der Gleitlagereinheit 32a an dem ersten Werkzeugelement 22a gleitend gelagert. Die Gleitlagereinheit 32a weist zumindest ein erstes Gleitlagerelement 34a auf. Die Gleitlagereinheit 32a weist zumindest ein zweites Gleitlagerelement 46a auf (vgl. Fig. 2). Das erste Gleitlagerelement 34a und das zweite Gleitlagerelement 46a sind zumindest teilweise korrespondierend zueinander ausgebildet. Das Gleitlagerelement 34a ist mit dem korrespondierenden zweiten Gleitlagerelement 46a zu einer Stabilisierung des zweiten Werkzeugelements 24a relativ zum ersten Werkzeugelement 22a vorgesehen. Das erste Gleitlagerelement 34a weist zumindest ein Schienenteil 58a auf. Der Schienenteil 58a des ersten Gleitlagerelements 34a ist einteilig mit dem ersten Werkzeugelement 22a verbunden (vgl. Fig. 4). Das zweite Gleitlagerelement 46a ist als Gleitteil 60a ausgebildet. Der Gleitteil 60a ist insbesondere dazu vorgesehen, auf dem Schienenteil 58a zu gleiten (vgl. Fig. 2). Die Gleitlagereinheit 32a weist ein weiteres erstes Gleitlagerelement 62a und ein korrespondierendes weiteres zweites Gleitlagerelement 64a auf. Die weiteren Gleitlagerelemente 34a, 46a sind in Form spezieller, aneinander angepasster Formkonturen des ersten Werkzeugelements 22a und des zweiten Werkzeugelements 24a ausgebildet. Durch die Formkonturen ist zumindest teilweise ein geometrischer Eingriff der Werkzeugelemente 22a, 24a bereitgestellt.

Die Gleitlagereinheit 32a ist zumindest teilweise einstückig mit der Verriegelungseinheit 90a ausgebildet. Die Gleitlagereinheit 32a ist zumindest teilweise einstückig mit dem Verriegelungselement 92a ausgebildet. Die Gleitführungsfläche 94a des Verriegelungselements 92a ist auch Teil der Gleitlagereinheit 32a.

In der zweiten Stellung des Koppelelements 26a relativ zum zweiten Werkzeugelement 24a ist eine von der Gleitlagereinheit 32a bereitgestellte Lagerung aufhebbar. Das Schienenteil 58a des Gleitlagerelements 34a weist einen schienenlosen Teil auf (vgl. Fig. 4). Das zweite Werkzeugelement 24a ist durch eine Bewegung entlang der Verschiebeachse 28a von dem ersten Werkzeugelement 22a lösbar. Das zweite Werkzeugelement 24a wird dabei in Richtung der Betätigungseinheit 70a bewegt. Der Gleitteil 60a des Gleitlagerelements 46a wird bei einem Bewegen des zweiten Werkzeugelements 24a relativ zum ersten Werkzeugelement 22a in den schienenlosen Teil geführt. Eine Lagerung des Gleitlagerelements 46a ist in dem schienenlosen Teil aufgehoben.

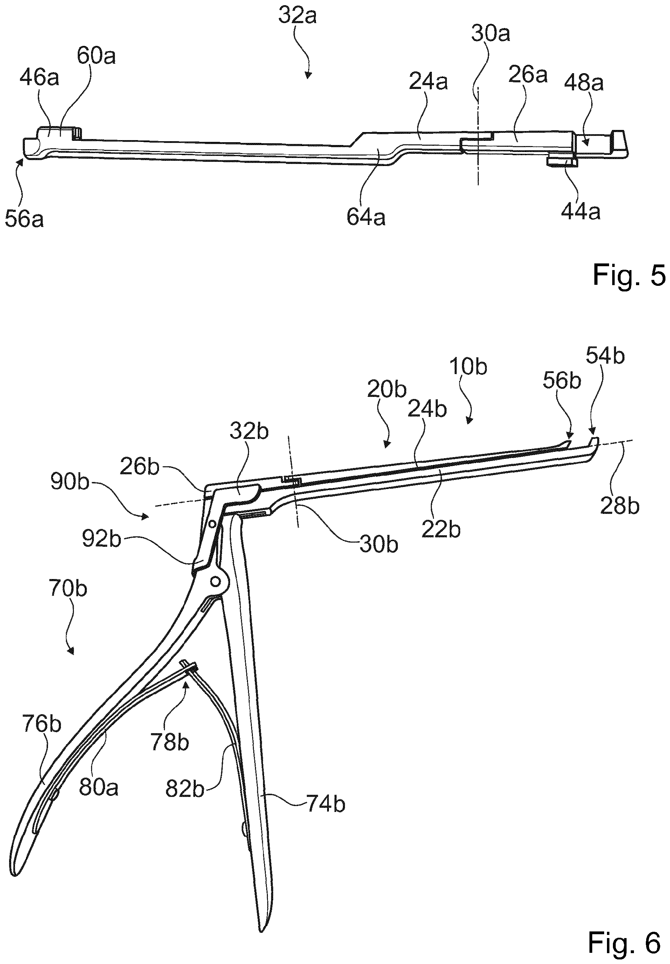

Die Gleitlagereinheit 32a lagert das Koppelelement 26a in der ersten Stellung gleitend am ersten Werkzeugelement 22a. Die Gleitlagereinheit 32a weist ein mit dem Koppelelement 26a verbundenes Führungsteil 44a auf (vgl. Fig. 5). Das Führungsteil 44a ist zu einer Stabilisierung des Koppelelements 26a vorgesehen. Das Führungsteil 44a ist zu einer Führung des zweiten Werkzeugelements 24a vorgesehen. Der Führungsteil 44a bildet eine Feder 40a für eine Nut-Feder-Verbindung. Das erste Werkzeugelement 22a weist eine Nut 52a zur Aufnahme des Führungsteils 44a auf. Die Nut 52a ist dazu vorgesehen ein Gleiten des Führungsteils 44a zu ermöglichen. Das erste Werkzeugelement 22a weist eine Öffnung 36a auf (vgl. Fig. 3). Die Öffnung 36a ist dazu vorgesehen, bei einem Schwenken des Koppelelements 26a von der ersten Stellung in die zweite Stellung zumindest teilweise das Führungsteil 44a freizugeben.

Bei einer Demontage des chirurgischen Instruments 10a wird das Koppelelement 26a von der ersten Stellung in die zweite Stellung relativ zum zweiten Werkzeugelement 24a zur Freigabe des Betätigungselements 72a überführt. In einem ersten Verfahrensschritt wird das Verriegelungselement 92a bewegt. Das Verriegelungselement 92a wird dabei gedreht. Das Verriegelungselement 92a gibt das Koppelelement 26a frei. In einem zweiten Verfahrensschritt wird das Koppelelement 26a um die Schwenkachse 30a bewegt. Das Führungsteil 44a wird bei einem Schwenken von der ersten Stellung des Koppelelement 26a in die zweite Stellung des Koppelelements 26a aus der Öffnung 36a geführt. In einem dritten Verfahrensschritt wird das zweite Werkzeugelement 24a relativ zum ersten Werkzeugelement 22a verschoben. Das zweite Werkzeugelement 24a wird zumindest im Wesentlichen in Richtung der Betätigungseinheit 70a verschoben. Das zweite Werkzeugelement 24a wird entlang der Verschiebeachse 28a verschoben. Eine Lagerung der Gleitlagereinheit 32a wird zumindest im Wesentlichen aufgehoben. In einem vierten Verfahrensschritt wird das zweite Werkzeugelement 24a von dem ersten Werkzeugelement 22a gelöst.

Zur Montage des chirurgischen Instruments 10a wird das zweite Werkzeugelement 24a in einem ersten Verfahrensschritt auf das erste Werkzeugelement 22a geschoben. Die Gleitlagereinheit 32a verbindet zumindest teilweise das erste Werkzeugelement 22a mit dem zweiten Werkzeugelement 24a. In einem zweiten Verfahrensschritt wird das Koppelelement 26a von der zweiten Stellung in die erste Stellung bewegt. Das Führungsteil 44a wird durch die Öffnung 36a geführt. In einem dritten Verfahrensschritt wird das Verriegelungselement 92a von der zweiten Stellung des Koppelelements 26a zur ersten Stellung des Koppelelements 26a zurück geschwenkt. Das Verriegelungselement 92a verhindert ein Schwenken des Koppelelements 26a.

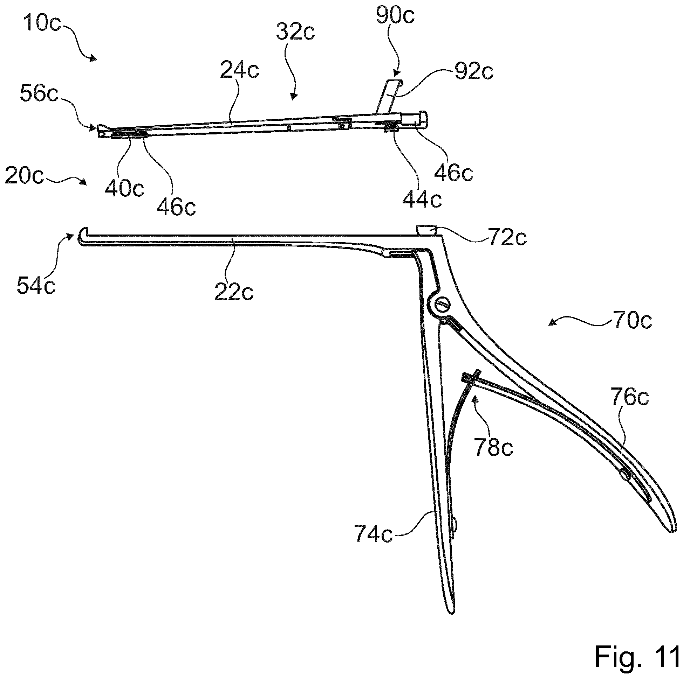

In den Fig. 6-11 sind zwei weitere Ausführungsbeispiele der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Fig. 1-5, verwiesen werden kann. Zur Unterscheidung der Ausführungsbeispiele ist der Buchstabe a den Bezugszeichen des Ausführungsbeispiels in der Fig. 1-5 nachgestellt. In den Ausführungsbeispielen der Figuren 6-11 ist der Buchstabe a durch die Buchstaben b und c ersetzt.

In den Figuren 6-8 ist ein weiteres chirurgisches Instrument 10b gezeigt. Das chirurgische Instrument 10b unterscheidet sich vom chirurgischen Instrument 10a im Wesentlichen durch eine Öffnungsrichtung einer Werkzeugeinheit 20b.

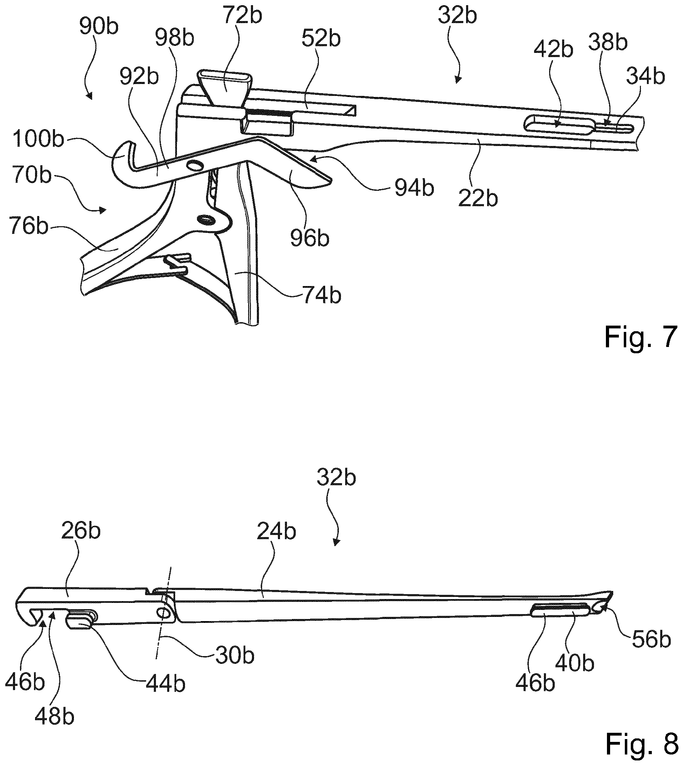

Ein zweites Werkzeugelement 24b der Werkzeugeinheit 20b ist zumindest im Wesentlichen stabförmig ausgebildet (vgl. Fig. 8). Die Werkzeugeinheit 20b weist ein erstes Schneideelement 54b auf. Die Werkzeugeinheit 20b weist ein zweites Schneideelement 56b auf. Das erste Schneideelement 54b ist mit einem ersten Werkzeugelement 22b der Werkzeugeinheit 20b einteilig ausgebildet. Das zweite Schneideelement 56b ist einteilig mit dem zweiten Werkzeugelement 24b ausgebildet. Das erste Schneideelement 54b und das zweite Schneideelement 56b begrenzen eine Öffnung. Die Öffnung ist dazu vorgesehen, ein zu bearbeitendes Objekt zwischen dem ersten Schneideelement 54b und dem zweiten Schneideelement 56b zu führen. Das erste Schneideelement 54b ist relativ zum zweiten Schneideelement 56b zumindest im Wesentlichen parallel angeordnet. Das erste Schneideelement 54b weist relativ zum ersten Werkzeugelement 22b einen Winkel zwischen 110° und 130° auf.

Die Werkzeugeinheit 20b weist eine Gleitlagereinheit 32b auf. Die Gleitlagereinheit 32b weist zumindest ein erstes Gleitlagerelement 34b auf (vgl. Fig. 7). Die Gleitlagereinheit 32b weist zumindest ein zweites Gleitlagerelement 46b auf (vgl. Fig. 8). Das erste Gleitlagerelement 34b ist zumindest teilweise als Nut 38b ausgebildet. Das zweite Gleitlagerelement 46b ist zumindest teilweise als Feder 40b ausgebildet. Das erste Gleitlagerelement 34b und das zweite Gleitlagerelement 46b stellen eine Feder-Nut-Verbindung bereit. Die Feder-Nut-Verbindung ist dazu vorgesehen, ein Gleiten des zweiten Werkzeugelements 24b relativ zum ersten Werkzeugelement 22b zu ermöglichen.

Zum Lösen des zweiten Werkzeugelements 24b vom ersten Werkzeugelement 22b weist die Gleitlagereinheit 32b eine weitere Nut 42b auf. Die weitere Nut 42b ist zur Lösung des zweiten Gleitlagerelements 46b vorgesehen. Die weitere Nut 42b ist zur Lösung der Nut-Feder-Verbindung vorgesehen. Die weitere Nut 42b schließt unmittelbar an die Nut 38b an. Die weitere Nut 42b ist zumindest 30 % breiter als die Nut 38b. Bei einem Zurückschieben des zweiten Werkzeugelements 24b relativ zum ersten Werkzeugelement 22b ist die Feder 40b in der weiteren Nut 42b angeordnet. Die weitere Nut 42b hebt eine Lagerung des ersten Gleitlagerelements 32b relativ zum zweiten Gleitlagerelement 46b auf.

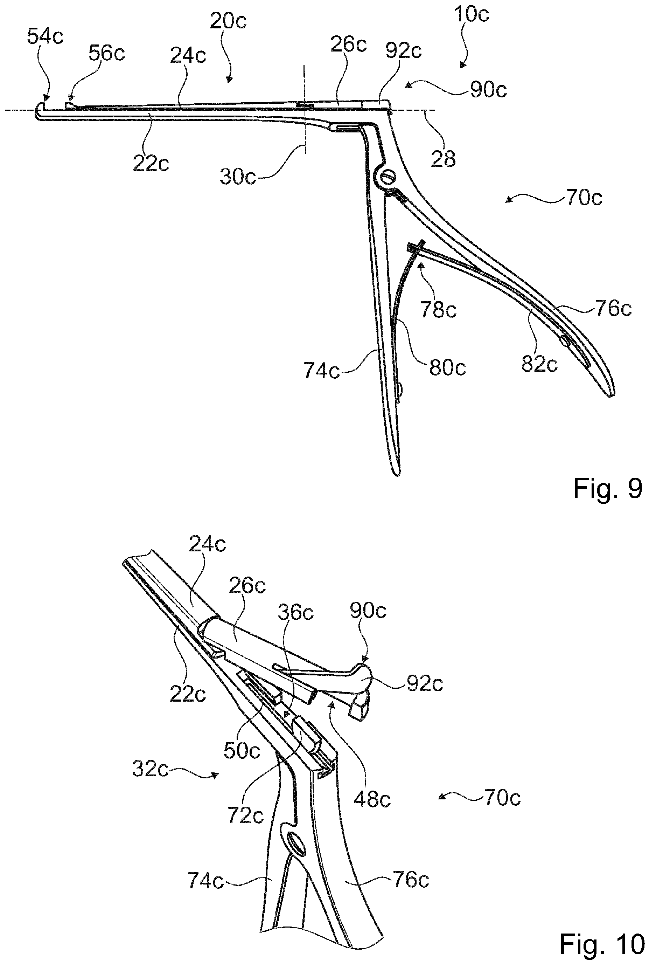

In den Figuren 9-11 ist ein weiteres chirurgisches Instrument 10c dargestellt.

Das chirurgische Instrument 10c weist eine Verriegelungseinheit 90c mit einem Verriegelungselement 92c auf. Das Verriegelungselement 92c ist an einem Koppelelement 26c einer Werkzeugeinheit 20c gelagert (vgl. Fig. 10). Das Verriegelungselement 92c ist mit dem Koppelelement 26c vernietet. Das Verriegelungselement 92c ist in einer zweiten Stellung des Koppelelements 26c relativ zu einem zweiten Werkzeugelement 24c der Werkzeugeinheit 20c entlang einer Verschiebeachse 28c beweglich gelagert. Eine Haupterstreckungsgerade des Verriegelungselements 92c ist zumindest in einer ersten Stellung des Koppelelements 26c relativ zum zweiten Werkzeugelement 24c zumindest im Wesentlichen parallel zu der Verschiebeachse 28c. Das Verriegelungselement 92c umschließt zumindest teilweise ein Betätigungselement 72c einer Betätigungseinheit 60c. Eine Schwenkachse des Verriegelungselements 92c ist senkrecht zu einer Schwenkachse 30c des Koppelelements 26c ausgerichtet. Die Schwenkachse des Verriegelungselements 92c ist in der ersten Stellung senkrecht zu einer Haupterstreckungsebene der Betätigungseinheit 70c. Bei einem Schwenken des Koppelelements 26c von der ersten Stellung des Koppelelements 26c zur zweiten Stellung des Koppelelements 26c um die Schwenkachse 30c bewegt sich das Verriegelungselement 92c zumindest teilweise mit.

Die Werkzeugeinheit 20c weist ein erstes Schneideelement 54c auf. Die Werkzeugeinheit 20c weist ein zweites Schneideelement 56c auf. Das erste Schneideelement 54c und das zweite Schneideelement 56c sind gemäß dem zweiten Ausführungsbeispiel, welches in den Figuren 6-9 gezeigt ist, ausgerichtet. In einem weiteren, nicht gezeigten, Ausführungsbeispiel, sind ein erstes Schneideelement und ein zweites Schneideelement gemäß dem ersten Ausführungsbeispiel, welches in den Figuren 1-5 gezeigt ist, ausgerichtet.

## Patentansprüche

1. Chirurgisches Instrument (10a; 10b; 10c) mit zumindest einer Werkzeugeinheit (20a; 20b; 20c), welche ein erstes Werkzeugelement (22a; 22b; 22c), zumindest ein zweites Werkzeugelement (24a; 24b; 24c) und zumindest ein mit dem zweiten Werkzeugelement (24a; 24b; 24c) verbundenes Koppelelement (26a; 26b; 26c) aufweist, welche gemeinsam relativ zu dem ersten Werkzeugelement (22a; 22b; 22c) entlang zumindest einer Verschiebeachse (28a; 28b; 28c) verschiebbar gelagert sind, und mit einer Betätigungseinheit (60a; 60b; 60c), welche zumindest ein Betätigungselement (72a; 72b; 72c) aufweist, welches in zumindest einer ersten Stellung des Koppelelements (26a; 26b; 26c) relativ zum zweiten Werkzeugelement (24a; 24b; 24c) mit dem Koppelelement (26a; 26b; 26c) gekoppelt ist, wobei das Koppelelement (26a; 26b; 26c) in zumindest einer zweiten Stellung relativ zum zweiten Werkzeugelement (24a; 24b; 24c) das Betätigungselement (72a; 72b; 72c) freigibt, **dadurch gekennzeichnet, dass** das Koppelelement (26a; 26b; 26c) um zumindest eine Schwenkachse (30a; 30b; 30c) schwenkbar an dem zweiten Werkzeugelement (24a; 24b; 24c) gelagert ist.

2. Chirurgisches Instrument (10a; 10b; 10c) nach Anspruch 1, **dadurch gekennzeichnet, dass** die Schwenkachse (30a; 30b; 30c) zumindest im Wesentlichen senkrecht zur Verschiebeachse (28a; 28b; 28c) ausgerichtet ist.

3. Chirurgisches Instrument (10a; 10b; 10c) nach Anspruch 1 oder 2, **dadurch gekennzeichnet, dass** die Schwenkachse (30a; 30b; 30c) zumindest im Wesentlichen parallel zu einer Haupterstreckungsebene der Betätigungseinheit (60a; 60b; 60c) ausgerichtet ist.

4. Chirurgisches Instrument (10a; 10b; 10c) nach einem der vorhergehenden Ansprüche, **dadurch gekennzeichnet, dass** die Werkzeugeinheit (20a; 20b; 20c) zumindest eine Gleitlagereinheit (32a; 32b; 32c) aufweist, welche in der ersten Stellung das zweite Werkzeugelement (24a; 24b; 24c) mit dem ersten Werkzeugelement (22a; 22b; 22c) verbindet und das zweite Werkzeugelement (24a; 24b; 24c) an dem ersten Werkzeugelement (22a; 22b; 22c) gleitend lagert.

5. Chirurgisches Instrument (10a; 10b; 10c) nach Anspruch 4, **dadurch gekennzeichnet, dass** in der zweiten Stellung eine von der Gleitlagereinheit (32a; 32b; 32c) bereitgestellte Lagerung aufhebbar und das zweite Werkzeugelement (24a; 24b; 24c) vom ersten Werkzeugelement (22a; 22b; 22c) lösbar ist.

6. Chirurgisches Instrument (10a; 10b; 10c) nach Anspruch 5, **dadurch gekennzeichnet, dass** das zweite Werkzeugelement (24a; 24b; 24c) durch eine Bewegung entlang der Verschiebeachse (28a; 28b; 28c) von dem ersten Werkzeugelement (22a; 22b; 22c) lösbar ist.

7. Chirurgisches Instrument (10a; 10b; 10c) nach einem der Ansprüche 4 bis 6, **dadurch gekennzeichnet, dass** die Gleitlagereinheit (32a; 32b; 32c) das Koppelelement (26a; 26b; 26c) in der ersten Stellung gleitend am ersten Werkzeugelement (22a; 22b; 22c) lagert.

8. Chirurgisches Instrument (10a; 10b; 10c) nach einem der vorhergehenden Ansprüche, **gekennzeichnet durch** zumindest eine Verriegelungseinheit (90a; 90b; 90c), welche das Koppelelement (26a; 26b; 26c) in der ersten Stellung verriegelt.

9. Chirurgisches Instrument (10a; 10b; 10c) nach Anspruch 8, **dadurch gekennzeichnet, dass** die Verriegelungseinheit (90a; 90b; 90c) zumindest ein relativ zur Werkzeugeinheit (20a; 20b; 20c) schwenkbar gelagertes Verriegelungselement (92a; 92b; 92c) aufweist.

10. Chirurgisches Instrument (10a; 10b) nach Anspruch 9, **dadurch gekennzeichnet, dass** das Verriegelungselement (92a; 92b) an dem ersten Werkzeugelement (22a; 22b) gelagert ist.

11. Chirurgisches Instrument (10c) nach Anspruch 9, **dadurch gekennzeichnet, dass** das Verriegelungselement (92c) an dem Koppelelement (26c) gelagert ist.

12. Chirurgisches Instrument (10a; 10b; 10c) zumindest nach den Ansprüchen 4 und 8, **dadurch gekennzeichnet, dass** die Gleitlagereinheit (32a; 32b) zumindest teilweise einstückig mit der Verriegelungseinheit (90a; 90b) ausgebildet ist.

13. Chirurgisches Instrument (10a; 10b) nach Anspruch 12, **dadurch gekennzeichnet, dass** das Verriegelungselement (92a; 92b) zumindest eine Gleitführungsfläche (94a; 94b) für das Koppelelement (26a; 26b) bereitstellt.

14. Verfahren zur Demontage eines chirurgischen Instruments (10a; 10b; 10c), insbesondere nach einem der vorhergehenden Ansprüche, mit zumindest einer Werkzeugeinheit (20a; 20b; 20c), welche ein erstes Werkzeugelement (22a; 22b; 22c), zumindest ein zweites Werkzeugelement (24a; 24b; 24c) und zumindest ein mit dem zweiten Werkzeugelement (24a; 24b; 24c) verbundenes Koppelelement (26a; 26b; 26c) aufweist, welche gemeinsam relativ zu dem ersten Werkzeugelement (22a; 22b; 22c) entlang zumindest einer Verschiebeachse (28a; 28b; 28c) verschiebbar gelagert sind, und mit einer Betätigungseinheit (60a; 60b; 60c), welche zumindest ein Betätigungselement (72a; 72b; 72c) aufweist, welches in zumindest einer ersten Stellung des Koppelelements (26a; 26b; 26c) relativ zum zweiten Werkzeugelement (24a; 24b; 24c) mit dem Koppelelement (26a; 26b; 26c) gekoppelt ist, wobei das Koppelelement (26a; 26b; 26c) um zumindest eine Schwenkachse (30a; 30b; 30c) schwenkbar an dem zweiten Werkzeugelement (24a; 24b; 24c) gelagert ist und wobei das Koppelelement (26a; 26b; 26c) von der ersten Stellung in zumindest eine zweite Stellung relativ zum zweiten Werkzeugelement (24a; 24b; 24c) zur Freigabe des Betätigungselements (72a; 72b; 72c) überführt wird.

## Claims

1. Surgical instrument (10a; 10b; 10c) with at least one tool unit (20a; 20b; 20c) comprising a first tool element (22a; 22b; 22c), at least one second tool element (24a; 24b; 24c) and at least one coupling element (26a; 26b; 26c) which is connected to the second tool element (24a; 24b; 24c), the at least one second tool element (24a; 24b; 24c) and the at least one coupling element (26a; 26b; 26c) being supported such that they are together displaceable relative to the first tool element (22a; 22b; 22c) along at least one displacement axis (28a; 28b; 28c),
and with an actuation unit (60a; 60b; 60c) comprising at least one actuation element (72a; 72b; 72c), which is coupled with the coupling element (26a; 26b; 26c) in at least one first position of the coupling element (26a; 26b; 26c) relative to the second tool element (24a; 24b; 24c),
wherein in at least one second position relative to the second tool element (24a; 24b; 24c), the coupling element (26a; 26b; 26c) releases the actuation element (72a; 72b; 72c),
**characterised in that**
the coupling element (26a; 26b; 26c) is supported on the second tool element (24a; 24b; 24c) pivotably around at least one pivot axis (30a; 30b; 30c).

2. Surgical instrument (10a; 10b; 10c) according to claim 1,
**characterised in that** the pivot axis (30a; 30b; 30c) is oriented at least substantially perpendicularly to the displacement axis (28a; 28b; 28c).

3. Surgical instrument (10a; 10b; 10c) according to claim 1 or 2,
**characterised in that** the pivot axis (30a; 30b; 30c) is oriented at least substantially parallel to a main extension plane of the actuation unit (60a; 60b; 60c).

4. Surgical instrument (10a; 10b; 10c) according to one of the preceding claims,
**characterised in that** the tool unit (20a; 20b; 20c) comprises at least one slide bearing unit (32a; 32b; 32c), which in the first position connects the second tool element (24a; 24b; 24c) to the first tool element (22a; 22b; 22c) and supports the second tool element (24a; 24b; 24c) in such a way that it is slidable on the first tool element (22a; 22b; 22c).

5. Surgical instrument (10a; 10b; 10c) according to claim 4,
**characterised in that** in the second position a support provided by the slide bearing unit (32a; 32b; 32c) can be undone and the second tool element (24a; 24b; 24c) can be released from the first tool element (22a; 22b; 22c).

6. Surgical instrument (10a; 10b; 10c) according to claim 5,
**characterised in that** the second tool element (24a; 24b; 24c) is releasable from the first tool element (22a; 22b; 22c) by a movement along the displacement axis (28a; 28b; 28c).

7. Surgical instrument (10a; 10b; 10c) according to one of claims 4 to 6,
**characterised in that** the slide bearing unit (32a; 32b; 32c) supports the coupling element (26a; 26b; 26c) in the first position in such a way that it is slidable on the first tool element (22a; 22b; 22c).

8. Surgical instrument (10a; 10b; 10c) according to one of the preceding claims,
**characterised by** at least one locking unit (90a; 90b; 90c), which locks the coupling element (26a; 26b; 26c) in the first position.

9. Surgical instrument (10a; 10b; 10c) according to claim 8,
**characterised in that** the locking unit (90a; 90b; 90c) comprises at least one locking element (92a; 92b; 92c) which is supported pivotably relative to the tool unit (20a; 20b; 20c).

10. Surgical instrument (10a; 10b) according to claim 9,
**characterised in that** the locking element (92a; 92b) is supported on the first tool element (22a; 22b).

11. Surgical instrument (10c) according to claim 9,
**characterised in that** the locking element (92c) is supported on the coupling element (26c).

12. Surgical instrument (10a; 10b; 10c) at least according to claims 4 and 8,
**characterised in that** the slide bearing unit (32a; 32b) is implemented at least partly integrally with the locking unit (90a; 90b)

13. Surgical instrument (10a; 10b) according claim 12,
**characterised in that** the locking element (92a; 92b) provides at least one slide guiding surface (94a; 94b) for the coupling element (26a; 26b).

14. Method for disassembling a surgical instrument (10a; 10b; 10c), in particular according to one of the preceding claims,
with at least one tool unit (20a; 20b; 20c) comprising a first tool element (22a; 22b; 22c), at least one second tool element (24a; 24b; 24c) and at least one coupling element (26a; 26b; 26c) which is connected to the second tool element (24a; 24b; 24c), the at least one second tool element (24a; 24b; 24c) and the coupling element (26a; 26b; 26c) being supported such that they are together displaceable relative to the first tool element (22a; 22b; 22c) along at least one displacement axis (28a; 28b; 28c),
and with an actuation unit (60a; 60b; 60c) comprising at least one actuation element (72a; 72b; 72c), which is coupled with the coupling element (26a; 26b; 26c) in at least one first position of the coupling element (26a; 26b; 26c) relative to the second tool element (24a; 24b; 24c),
wherein the coupling element (26a; 26b; 26c) is supported on the second tool element (24a; 24b; 24c) pivotably around at least one pivot axis (30a; 30b; 30c) and wherein for a release of the actuation element (72a; 72b; 72c) the coupling element (26a; 26b; 26c) is transferred from the first position into at least one second position relative to the second tool element (24a; 24b; 24c).

## Revendications

1. Instrument chirurgical (10a ; 10b ; 10c) avec au moins une première unité d'outil (20a ; 20b ; 20c) comprenant un premier élément d'outil (22a ; 22b ; 22c), au moins un deuxième élément d'outil (24a ; 24b ; 24c) et au moins un élément de couplage (26a ; 26b ; 26c) lié au deuxième élément d'outil (24a ; 24b ; 24c), l'élément de couplage (26a ; 26b ; 26c) et le deuxième élément d'outil (24a ; 24b ; 24c) étant supportés de manière à être conjointement déplaçables par rapport au premier élément d'outil (22a ; 22b ; 22c) le long d'un axe de déplacement (28a ; 28b ; 28c),
et avec une unité d'actionnement (60a ; 60b ; 60c) comprenant au moins un élément actionneur (72a ; 72b ; 72c) couplé à l'élément de couplage (26a ; 26b ; 26c) dans au moins une première position de l'élément de couplage (26a ; 26b ; 26c) par rapport au deuxième élément d'outil (24a ; 24b ; 24c), l'élément de couplage (26a ; 26b ; 26c) relâchant l'élément actionneur (72a ; 72b ; 72c) dans au moins une deuxième position par rapport au deuxième élément d'outil (24a ; 24b ; 24c),
**caractérisé en ce que** l'élément de couplage (26a ; 26b ; 26c) est supporté sur le deuxième élément d'outil (24a ; 24b ; 24c) de manière à être pivotable autour d'au moins un axe de pivotement (30a ; 30b ; 30c).

2. Instrument chirurgical (10a ; 10b ; 10c) selon la revendication 1,
**caractérisé en ce que** l'axe de pivotement (30a ; 30b ; 30c) est orienté au moins sensiblement perpendiculairement à l'axe de déplacement (28a ; 28b ; 28c).

3. Instrument chirurgical (10a ; 10b ; 10c) selon la revendication 1 ou 2,
**caractérisé en ce que** l'axe de pivotement (30a ; 30b ; 30c) est orienté au moins sensiblement en parallèle à un plan d'étendue principale de l'unité d'actionnement (60a ; 60b ; 60c).

4. Instrument chirurgical (10a ; 10b ; 10c) selon l'une des revendications précédentes,
**caractérisé en ce que** l'unité d'outil (20a ; 20b ; 20c) comprend au moins une unité palier-lisse (32a ; 32b ; 32c), qui dans la première position lie le deuxième élément d'outil (24a ; 24b ; 24c) avec le premier élément d'outil (22a ; 22b ; 22c) et supporte le deuxième élément d'outil (24a ; 24b ; 24c) au premier élément d'outil (22a ; 22b ; 22c) d'une manière glissante.

5. Instrument chirurgical (10a ; 10b ; 10c) selon la revendication 4,
**caractérisé en ce que** dans la deuxième position un support pourvu par l'unité palier-lisse (32a ; 32b ; 32c) peut être défait et le deuxième élément d'outil (24a ; 24b ; 24c) peut être détaché du premier élément d'outil (22a ; 22b ; 22c).

6. Instrument chirurgical (10a ; 10b ; 10c) selon la revendication 5,
**caractérisé en ce que** le deuxième élément d'outil (24a ; 24b ; 24c) est détachable du premier élément d'outil (22a ; 22b ; 22c) par un mouvement le long de l'axe de déplacement (28a ; 28b ; 28c).

7. Instrument chirurgical (10a ; 10b ; 10c) selon l'une des revendications 4 à 6,
**caractérisé en ce que** l'unité palier-lisse (32a ; 32b ; 32c) supporte l'élément de couplage (26a ; 26b ; 26c) dans la première position d'une manière glissante au premier élément d'outil (22a ; 22b ; 22c).

8. Instrument chirurgical (10a ; 10b ; 10c) selon l'une des revendications précédentes,
**caractérisé par** au moins une unité de verrouillage (90a ; 90b ; 90c) verrouillant l'élément de couplage (26a ; 26b ; 26c) dans a première position.

9. Instrument chirurgical (10a ; 10b ; 10c) selon la revendication 8,
**caractérisé en ce que** l'unité de verrouillage (90a ; 90b ; 90c) comprend au moins un élément de verrouillage (92a ; 92b ; 92c) supporté pivotablement par rapport à l'unité d'outil (20a ; 20b ; 20c).

10. Instrument chirurgical (10a ; 10b) selon la revendication 9,
**caractérisé en ce que** l'élément de verrouillage (92a ; 92b) est supporté au premier élément d'outil (22a ; 22b).

11. Instrument chirurgical (10c) selon la revendication 9,
**caractérisé en ce que** l'élément de verrouillage (92c) est supporté à l'élément de couplage (26c).

12. Instrument chirurgical (10a ; 10b ; 10c) au moins selon les revendications 4 et 8,
**caractérisé en ce que** l'unité palier-lisse (32a ; 32b) est réalisée au moins partiellement intégralement avec l'unité de verrouillage (90a ; 90b).

13. Instrument chirurgical (10a ; 10b) selon la revendication 12,
**caractérisé en ce que** l'élément de verrouillage (92a ; 92b) fournit au moins une surface à guidage-glissant (94a ; 94c) pour l'élément de couplage (26a ; 26b).

14. Procédé pour désassemblage d'un instrument chirurgical (10a ; 10b ; 10c), en particulier selon l'une des revendications précédentes,
avec au moins une unité d'outil (20a ; 20b ; 20c) comprenant un premier élément d'outil (22a ; 22b ; 22c), au moins un deuxième élément d'outil (24a ; 24b ; 24c) et au moins un élément de couplage (26a ; 26b ; 26c) lié au deuxième élément d'outil (24a ; 24b ; 24c), l'élément de couplage (26a ; 26b ; 26c) et le deuxième élément d'outil (24a ; 24b ; 24c) étant supportés de manière à être conjointement déplaçables par rapport au premier élément d'outil (22a ; 22b ; 22c) le long d'un axe de déplacement (28a ; 28b ; 28c),
et avec une unité d'actionnement (60a ; 60b ; 60c) comprenant au moins un élément actionneur (72a ; 72b ; 72c) couplé à l'élément de couplage (26a ; 26b ; 26c) dans au moins une première position de l'élément de couplage (26a ; 26b ; 26c) par rapport au deuxième élément d'outil (24a ; 24b ; 24c),
où l'élément de couplage (26a ; 26b ; 26c) est supporté sur le deuxième élément d'outil (24a ; 24b ; 24c) de manière à être pivotable autour d'au moins un axe de pivotement (30a ; 30b ; 30c) et
où l'élément de couplage (26a ; 26b ; 26c) est transféré de la première position dans au moins une deuxième position par rapport au deuxième élément d'outil (24a ; 24b ; 24c) pour un relâchement de l'élément actionneur (72a ; 72b ; 72c).
